# EUROPEAN PATENT APPLICATION

(11) **EP 4 234 709 A1**
(43) Date of publication of application: **30.08.2023**
(21) Application number: 23158370.9
(22) Date of filing: 23.02.2023
(51) Int. Cl.: C12P 19/04, C08L 1/02

(54) **A FOOD WASTE EXTRACT FOR CULTIVATION OF MICROORGANISMS AND PRODUCTION OF BACTERIAL CELLULOSE AND A PROCESS FOR OBTAINING SAID EXTRACT**

(30) Priority: 28.02.2022 SI 202200026
(71) Applicant: Univerza v Mariboru, 2000 Maribor (SI)
(72) Inventor: Gorgieva, Selestina, 2000 Maribor (SI); Trcek, Janja, 2000 Maribor (SI); Osmic, Azra, 2000 Maribor (SI); Cepec, Eva, 2000 Maribor (SI)
(74) Representative: Patentni Biro AF d.o.o.

(57) **Abstract**

The invention belongs to the field of microbiology, more precisely to the field of production of various microbial products such as bacterial cellulose. The present invention relates to an extract from food wastes for the cultivation of microorganisms and a process for obtaining said extract. The essence of the invention is that the source material for preparation of a novel growth medium is grape pomace, said pomace obtained after squeezing fluid from fruit and comprising mostly envelopes and seeds, which are processed by the following steps:
a) lyophilisation of grape pomace,
b) grinding of grape pomace lyophilised in step a) with any suitable device, preferably with a pulveriser,
c) addition of water to grape pomace ground in step b), wherein the maximal final pomace concentration is 30%,
d) autoclaving the mixture obtained in step c), at a temperature of at least 121 °C and a pressure of 1.1 bar, wherein the duration is at least 20 min with the possibility of time reduction if autoclaving is performed at a higher temperature,
e) separation of solid particles from the fluid part of the mixture autoclaved in step d), such as decantation, sedimentation, centrifugation, filtration or draining in order to obtain a liquid medium or an extract, respectively, which is suitable for use as a growth medium for microorganisms.

## Description

### Field of the invention

The present invention belongs to the field of microbiology, more precisely to the field of cultivation of microorganisms, particularly bacteria, as well as to the production of various microbial products such as bacterial cellulose. The present invention relates to an extract from food waste for cultivation of microorganisms and production of bacterial cellulose and a process for obtaining said extract. The invention also covers a method of producing bacterial cellulose, wherein the extract from food wastes is used as a growth medium.

### Background of the invention and the technical problem

Polysaccharides are polymeric structures of carbohydrates, in which individual units of mono and di-saccharides are bound together by glycosidic linkages. One of such polysaccharides is cellulose, a natural polymer comprising glucose molecules. Most known cellulose is plant-derived cellulose; however, numerous bacterial species are able to synthesise cellulose, most notably bacteria from genera *Acetobacter, Azotobacter, Rhizobium, Pseudomonas, Salmonella, Alcaligenes, Agrobacterium* and *Sarcina.* Production of bacterial cellulose (also termed nanocellulose) depends on several factors, such as the type of growth medium, environmental factors such as pH, temperature and dissolved oxygen, as well as formation of side products such as gluconic acid, which hampers bacterial cellulose synthesis. The growth medium usually comprises a carbon source, a nitrogen source and other macro- and micronutrients, wherein cellulose production is generally best when bacteria are supplied with abundant carbon sources and minimal amounts of nitrogen sources. Carbon sources are usually glucose and sucrose, while less frequently fructose, maltose, xylose, starch, glycerol and molasses are used. One of the standard growth media is Hestrin Schramm (HS) medium, which comprises 20 g/L glucose, 5 g/L peptone, 5 g/L yeast extract, 1.15 g/L citric acid, 2,7 g/L Na₂HPO₄, pH 6.0, The addition of amino acids, ethanol or some other acids may increase the amount of produced cellulose.

The properties of bacterial cellulose differ from the plant cellulose, wherein bacterial cellulose is pure, strong, with high water retention capacity and is very mouldable. By adapting cultivation method or controlling bacterial cellulose synthesis, respectively, some properties of produced cellulose may be controlled, which is particularly required for uses in the field of medicine, food science, cosmetics, paper production as well as textile industry (Gorgieva and Trček, 2019; doi: 10.3390/nano9101352). Bacterial cellulose is often used as a thickening agent and a stabiliser in food products, while it can also be used with the aim to reduce calories in dietary products. Microbial cellulose also plays a role in product packaging, particularly food, in order to avoid or reduce use of plastic packaging. In the field of medicine, the most interesting use of bacterial cellulose is wound treatment in case of burns and as implants or as replacement structures for the cardiovascular system, digestive tract, and urinary tract (Swingler et al., 2021, doi: 10.3390/polym13030412; Molina de Olyveira et al., 2016, doi: 10.1016/B978-0-323-42865-1.00003-9).

Due to suboptimal efficiency of procedures for the production of bacterial cellulose, the price of the latter is still too high to be sustainable for wider use, hence the development is directed into the optimisation of bacterial cultivation, search for new bacterial species and strains (as described in patent RU2681281C1, for example), which show a high production of cellulose, as well as into search of new growth media that are able to stimulate cellulose synthesis.

Therefore, the technical problem, which is solved by the present invention, is development of a new growth medium that will enable growth of bacteria capable of cellulose production as well as large amounts of produced cellulose. The aim of the invention is that the growth medium is based on food waste that is highly abundant and low-cost source. A further purpose of the invention is that the growth medium does not need any additional components of standard media, such as carbon sources, nitrogen sources, micro- and macronutrients and similar.

### Prior art

Use of food waste in the field of preparation of microbial growth media is generally known, however, the methods for processing said food waste differ significantly.

Patent application WO2021087738 relates to a method of preparation of bacterial cellulose, wherein the growth medium was a soy hydrolysate, which is formed during enzymatic production of soy oil. Patent application JP2009232813A describes use of food waste in soy processing (okara), which is then enzymatically hydrolysed and used for cultivation of bacteria. Waste of soy processing has also been used in the solution disclosed in document CN108977478, wherein the hydrolysate was prepared by drying the waste, grinding it into powder and adding dilute sulfuric acid. The acid hydrolysis was performed at high temperature, and then the resulting hydrolysate was filtered. In this form, it was added to the other components of the growth medium and the pH was adjusted accordingly.

Patent application CN110205349 describes the use of hydrolysate in the preparation of bacterial cellulose, wherein side products of rice processing have been used, particularly the outer rice envelope. The method of obtaining bacterial cellulose comprises the following steps:
- mechanical pre-processing of rice envelopes,
- enzyme treatment for preparation of a hydrolysate,
- fermentation of the obtained hydrolysate,
- sterilisation of bacterial cellulose, and
- purification of obtained bacterial cellulose.

In addition to soy and rice other sources have been used as source material, such as apples (CN104031956A), citrus (CN102168056A) and grapes (CN110295133A and CN109055457A). Patent application CN109055457A describes a method of preparation of growth medium supplement prepared from grapes, wherein the method comprises a step of enzymatic hydrolysis, which is not preferred due to costs connected with enzymes as well as due to the need for removal of used enzymes.

References describing the preparation of microbial growth media without the use of enzymes are rare. For example, Cerrutti et al. (2015; doi: 10.1 002/app.431 09) used a growth medium with an extract of the grape pulp as a carbon source and corn steep liquor as a nitrogen source for cultivation of *Gluconacetobacter xylinus.* The seeds and stems from Semillon grapes pressed for wine-making were separated and the remaining skins and residual pulp were washed three times with distilled water and then homogenised in a Waring blender using a minimum required volume of 0.10 M citric acid-sodium citrate buffer at pH 5.0. The grape extract obtained was filtered through a cloth and centrifuged. The clarified liquid filtrate was frozen at -20 °C until used.

Ogrizek et al. (2021; doi: 10.3390/pr9071088) describe the use of waste from wine industry in production of bacterial cellulose with the aim to avoid use of costly commercially available media. Red and white grape bagasse was used as a carbon source replacement and the sole nutrient in the medium. White grape pomace was prepared in the following manner: immediately after de-stemming and crushing, the mash was pressed with the press to a maximum pressure of 1.5 bar. After cooking at 70 °C with distilled water, the mash was homogenised in a blender, which was followed by sieving to obtain an almost fluid mash. The procedure for red grapes was the same, and the only difference was that after de-stemming and crushing, 12-days of maceration with inoculated alcoholic fermentation followed. The measured pH of the white bagasse extract was 3.80 and the red extract 3.50. Obtained cellulose had improved water holding capacity, and the produced BC films exhibited improved flexibility when compared to bacterial cellulose obtained in standard HS medium.

All described methods differ from the present invention in the manner of preparation of the extract from food waste.

### Description of the solution to the technical problem

The essence of the invention is in that the source material for the preparation of novel growth medium is grape pomace (grape marc, grape bagasse), which are formed after extraction of fluids from fruit and mostly comprise envelope and seeds, which are processed in the following manner:
a) lyophilisation of grape pomace, preferably for 24 to 72 hours at a temperature from -10 °C to -90 °C, and pressure 1 mbar, or any combination of temperature and pressure which enables a direct sublimation of ice without any intermediate thawing, wherein the final moisture of pomace is from 5 % to 15 %, preferably around 7 %,
b) grinding of lyophilised pomace obtained in step a) with any suitable device, wherein the preferred choice is pulverisation at 1000 rpm to 6000 rpm, from 1 min to 5 min, and sieve size from 0.08 µm to 1 µm,
c) addition of water to grinded pomace obtained in step b) up to maximal final pomace concentration of 30 %, preferably from 5 to 15 %,
d) autoclaving the mixture obtained in step c), at a temperature of at least 121 °C, pressure 1.1 bar, and duration of 20 min with the possibility of time reduction for example for 5 to 10 min at higher temperature (for example 134 °C),
e) separation of solid particles from the fluid part, such as decantation, sedimentation, centrifugation, filtration or simple draining of the mixture autoclaved in step d) in order to obtain a fluid medium or an extract, respectively, which is suitable for use as a growth medium for microorganisms, wherein the preferred choice is filtration through 0.45 um polytetrafluoroethylene (PTFE) filter.

Grape pomace can be any variety or a mixture of different varieties, especially the Chardonnay or Pinot Blanc.

Lyophilisation enables that the pomace is quickly frozen, which prevents further spoiling or changing of the pomace, which could affect the composition of the extract. It may be carried out in the following conditions: at temperature up to -90 °C, pressure 1mbar, and duration of 48 hours, wherein the final pomace moisture is preferably 7 %. After lyophilisation, grinding is performed with any suitable device, preferably with a mill or a pulveriser. Suitably fine particles are obtained by an important step of grinding that enables efficient extraction of compounds from grape pomace, which can be used in the final extract by microorganisms as a carbon source, nitrogen source, micronutrients, etc. Autoclaving or thermal processing, respectively, causes degradation of polymers, which may also be used by microorganisms for growth and synthesis of products.

The medium or the extract from grape pomace obtained according to the above-described method may be used in cultivation of microorganisms, particularly bacteria. Bacteria do not require any other component; hence, the final filtrate represents an independent growth medium.

The extract according to the invention may be further provided with other additives or supplements, such as
- ethanol,
- glycerol,
- mannitol,
- acetic acid or other acids,
- different carbon sources, such as glucose, sucrose, fructose, maltose, xylose, starch, mannose, glycerol and molasses,
- amino acids,
- yeast extract,
- peptone,
- various vitamins and minerals,
- HS medium (Hestrin Schramm),
- RAE medium with different content of ethanol and acetic acid (in general, RAE medium comprises glucose, peptone, yeast extract, citric acid, Na₂HPO₄, acetic acid, ethanol and water),
or any combination of the above-mentioned additives.

The preferred additives are ethanol, acetic acid and/or RAE medium.

The so obtained medium from grape pomace may be used for cultivation of microorganisms, particularly bacteria. The extract according to the invention is particularly useful for production of extracellular polysaccharides, mostly bacterial cellulose (nanocellulose), wherein the extract may be used for growth of selected bacteria from the group consisting of genera *Acetobacter, Acidomonas, Alcaligenes, Agrobacterium, Ameyamaea, Asaia, Azotobacter, Bombella, Commensalibacter, Endobacter, Entomobacter, Gluconacetobacter, Komagataeibacter, Kozakia, Neoasaia, Neokomagataea, Nguyenibacter, Pseudomonas, Rhizobium, Saccharibacter, Salmonella, Sarcina, Swaminathania, Swingsia* and *Tanticharoenia.*

Use of the process for obtaining the extract from grape pomace and use of the extract is preferably in production of bacterial extracellular polysaccharides, preferably in production of cellulose.

The process for the preparation of bacterial cellulose using the extract according to the invention comprises the following steps:
a) preparation of the extract and optionally addition of additives in order to obtain a growth medium,
b) cultivation of the selected bacterial strain from any bacterial genera defined above in a suitable amount of growth medium obtained in step a), at temperature from 25 °C to 32 °C, preferably 30 °C, for at least 24 hours with shaking, followed by at least 3 days without shaking and at the same temperature to obtain a pellicle or cellulose membrane at the surface of the growth medium,
c) removal of the pellicle obtained in step b) from the medium and cooking in 0.5 M NaOH at temperature of 80 °C for 30 minutes at constant mixing,
d) washing the cellulose membrane obtained in step c) in distilled water, and
e) drying the cellulose membrane washed in step d).

Use of the bacterial cellulose obtained with the extract or growth medium comprising the extract according to the invention in food industry, medicine, cosmetics, paper industry and textile industry.

The food waste extract for the cultivation of microorganisms and production of bacterial cellulose and a process for obtaining said extract according to the invention will be further described on the basis of examples and figures, which show:
- Figure 1: HPLC analysis of sugars from grape pomace (hydrolysate). Hydrolysate 4 is made from 5.5 % pomace and hydrolysate 25 from 10% pomace, both of variety Pinot Blanc.
- Figure 2: Dry weight (g) of produced bacterial cellulose by *Komagataeibacter melomenusus* in hydrolysate (H), and addition of complex RAE medium, 1% ethanol (ET) and 1% acetic acid (OK). Hydrolysate 4 is made with 5.5 % pomace and hydrolysate 25 from 10% pomace, both of variety Pinot Blanc.
- Figure 3: Wet wight (g) of produced bacterial cellulose by *Komagataeibacter melomenusus* in hydrolysate (H), and addition of complex RAE medium, 1% ethanol (ET) and 1% acetic acid (OK). Hydrolysate 4 is made with 5.5 % pomace and hydrolysate 25 from 10% pomace, both of variety Pinot Blanc.
- Figure 4: FTIR-curve of the bacterial cellulose produced by *K. melomenusus* in the medium with grape pomace extract.
- Figure 5: Scanning electron microscopy (SEM) image of the bacterial cellulose produced by *K. melomenusus* in the medium with grape pomace extract.
- Figure 6: Results of analysis of crystallinity with X-ray diffraction for bacterial cellulose produced by *K. melomenusus* in the medium with grape pomace extract.

### Examples

### Example 1 - Extract preparation

Grape pomace of Chardonnay and Pino Blanc were used, said pomace resulting from squeezing of liquid from fruits and mostly comprise envelopes and seeds, which were processed with the following steps:
a) lyophilisation of grape pomace for 48 hours, at temperature -90 °C, and pressure 1 mbar, wherein the final moisture of pomace was 7 %,
b) grinding in step a) lyophilised pomace with a pulveriser at 6000 rpm, for 5 min, and sieve size of 0.5 µm,
c) addition of water to ground pomace obtained in step b) up to final pomace concentration of 5.5 % or 10 %,
d) autoclaving the mixture obtained in step c), at temperature of 121 °C, pressure 1.1 bar, for 20 min,
e) separation of solid particles from the fluid part of the mixture autoclaved in step d) in order to obtain a fluid medium or an extract, respectively, which is suitable for use as a growth medium for microorganisms, with filtration through a 0.45 um polytetrafluoroethylene (PTFE) filter.

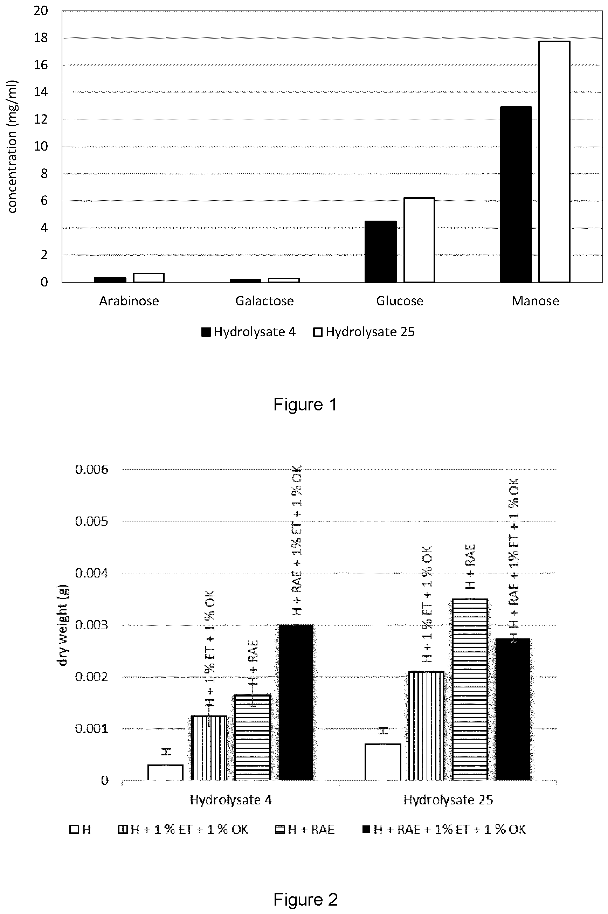

The prepared extract was analysed with HPLC. Figure 1 shows the results of HPLC analysis of sugars from the extract prepared with 5.5 % pomace (black columns) and from 10 % pomace (white columns), both from the variety Pinot Blanc. Detected sugars were arabinose and galactose in lower concentrations and mostly glucose and mannose, wherein the sugar concentration was, as expected, higher in the case of 10 % pomace. Presence of the following sugars was in the following amounts:
- arabinose and galactose in amount lower than 1 mg/mL, i.e., 0.3 and 0.7 mg/mL for arabinose and 0.2 and 0.3 mg/mL for galactose;
- glucose in amount higher than 4 mg/mL, i.e., 4.5 and 6.2 mg/mL; and
- mannose in amount higher than 12 mg/mL, i.e., 12.9 and 17.8 mg/mL.

In the extract prepared with 5.5 % pomace, 72.5 % of all measured sugars was mannose, 25% glucose, 1% galactose, 1.5% arabinose. In the extract prepared with 10 % pomace, 71.2 % of all measured sugars was mannose, 24.8% glucose, 1.2 % galactose, 2.8 arabinose. Hence, approximately one quarter of sugars is glucose, mannose is approximately 3-fold more abundant, while galactose and arabinose represent 1 to 3 % of all sugars.

The so prepared extract from pomace of Pinot Blanc was used for cultivation of bacterial strain *K. melomenusus.*

### Example 2 - Preparation of bacterial cellulose

Bacterial strain *K. melomenusus* were kept at -80 °C in the presence of cryoprotectant until use. The process was initiated with inoculation on a solid RAE medium comprising 1 vol% of ethanol and 1 vol% of acetic acid. The growth medium with inoculated strain was incubated in a moist atmosphere for 3 days (suitable time interval from 2 to 7 days) at 30 °C (suitable temperature range from 25 °C do 32 °C). One bacterial colony has been transferred into 5 ml of sterile extract into 13 ml sterile test tube with a cover (suitable are smaller or larger volumes of growth media and vessels, such as Erlenmeyer flasks and bioreactors) and shaken for 24 hours (suitable is longer shaking if the strain is to be more abundant) at 180 rpm and 30 °C. The shaker was then stopped, and the flasks were further incubated in a static manner at 30 °C. After three days (the time of static incubation may be prolonged for few days, up to cca. 3 weeks), the pellicle formed at the surface of the medium was removed and cooked in 0.5 M NaOH at 80 °C for 30 min at constant mixing. Afterwards, the membrane was washed in distilled water until the pH of distilled water. The so processed cellulose membranes were dried at room temperature.

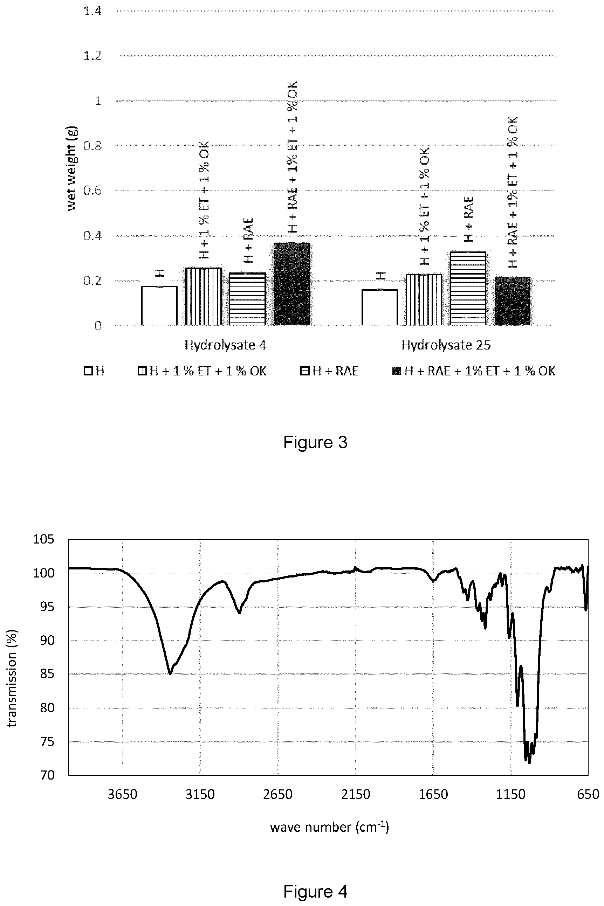

Figures 2 and 3 show dry and wet weight of the bacterial cellulose produced by *K. melomenusus* in the following media:
- in the extract as prepared in example 1 (H4 or H25),
- in the extract, as prepared in example 1 with the addition of 1% ethanol (ET) and 1% acetic acid (OK),
- in the extract, as prepared in example 1 with the addition of RAE medium, and
- in the extract, as prepared in example 1 with the addition of 1% ethanol (ET) and 1% acetic acid (OK) and RAE medium.

The dry weight (g) of produced bacterial cellulose was highest in the extract with additives and similar is valid also for the wet weight, wherein the differences in wet weight are smaller. The highest quantities of produced cellulose were in the case of RAE medium with or without ethanol and acetic acid.

### Example 3 - Characterisation of produced cellulose

In example 2 obtained cellulose was characterised with FTIR spectroscopy, X-ray diffraction (XRD) and SEM microscopy. FTIR spectroscopic analysis of the bacterial cellulose membrane shown in figure 4 shows characteristic absorption bands for cellulose: a signal in the area of 3200-3600 cm⁻¹, which corresponds to intra-molecular O-H bonds in the hydroxyl group, a band at 2890 cm⁻¹ with a peak at 2870 cm⁻¹, which corresponds to CH and CH₂ stretching vibrations, a band at 1355 cm⁻¹, which is characteristic for symmetric stretching vibrations of CH₂ and bands in the area of 1170-1000 cm⁻¹, which correspond to O-H, C-H and C-O-C stretching vibrations.

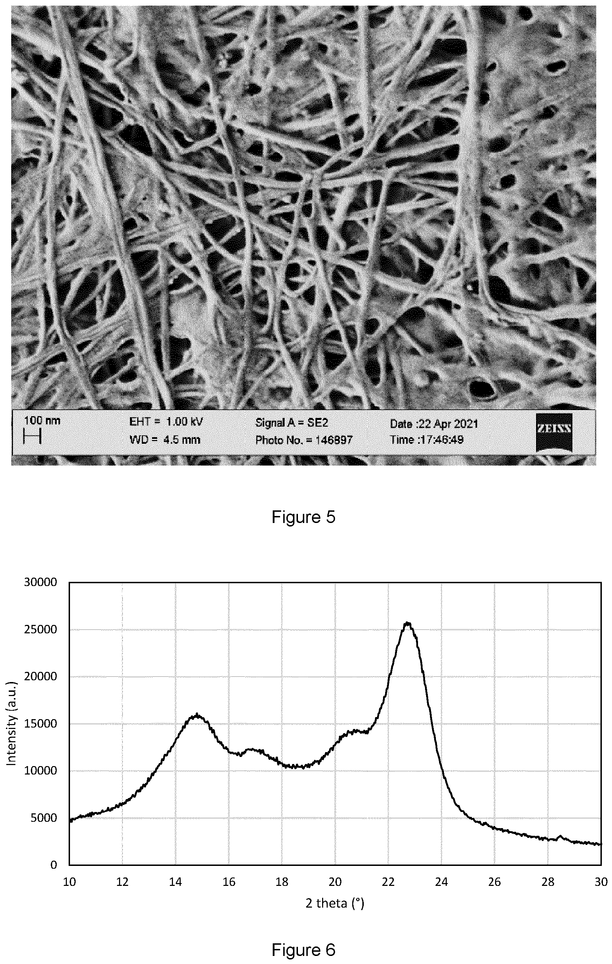

Figure 5 shows a SEM image of the upper surface of the bacterial cellulose membrane. A typical, intertwined mesh structure with isotropic nanofibers with intermediate pores is clearly visible.

XRD analysis of the bacterial cellulose was performed with an X-ray diffractometer D2 Phaser (Bruker, Germany). Using radiation Cu-kα at 30 kV and 10 mA (α=1.54439 Å) the diffraction angle 2θ between 5° and 30° with step 0.030° and 0.2 s/step was measured. Crystallinity, which is in this case 71.5%, is calculated on the basis of ratio between the integrated surface of crystalline peaks and the integrated total surface of the diffraction spectre. Crystallinity affects the mechanical properties, hardness and rigidity of cellulose and materials obtained from cellulose. Higher crystallinity of cellulose causes an increase in the Young module, tensile strength, density and hardness. The literature cites main diffraction peaks of bacterial cellulose at 2θ=14.7°, 16.9° and 22.8°, which are contributed to the interplanar distance characteristic for I_{α} and I_{β} basic cellulose crystal structure (100 I_{α}, 110I_{β} and 010 I_{β} relate to angle 15° and 110 I_{α} and 200 I_{β} to angle 22.5 °). The diffractogram shown in figure 6 shows that peaks at 2θ angle 14.7 ° and 23.0 ° are present, while the peak at 16.9 ° has very small intensity and is not distinct. A further two additional peaks at 2θ = 20.8° and 9.8° can be seen. The peak at 2θ=20.8° could be attributed to cellulose II, but this cellulose II has a characteristic peak at angle 12,1°, which was not detected. Even if cellulose II is present, the intensity of diffraction planes indicates that cellulose I is prevailing (higher intensity at diffraction plane 14.7° and 23.0° in comparison to 20.8°).

## Claims

1. An extract from food waste for the cultivation of bacteria and/or production of extracellular polysaccharides, particularly bacterial cellulose, **characterised in that** the extract is obtained with the following steps:
a) lyophilisation of grape pomace,
b) grinding of grape pomace lyophilised in step a),
c) addition of water to grape pomace ground in step b), wherein the maximal final pomace concentration is 30%,
d) autoclaving the mixture obtained in step c), and
e) separation of solid particles from the fluid part in order to obtain a liquid medium wherein these steps enable presence of the following sugars in the following amounts:
- arabinose and galactose in amount lower than 1 mg/mL and 0.5 mg/mL, respectively; and
- glucose and mannose in amount higher than 4 mg/mL and 12 mg/mL, respectively.

2. The extract according to claim 1, **characterised in that** the pomace is of any variety or mixture of varieties, preferably of variety Chardonnay or Pinot Blanc.

3. The extract, according to claim 1 or claim 2, **characterised in that** it is supplemented with further additives such as:
- ethanol,
- glycerol,
- mannitol,
- acetic acid or other acids,
- different carbon sources, such as glucose, sucrose, fructose, maltose, xylose, starch, mannose, glycerol and molasses,
- amino acids,
- yeast extract,
- peptone,
- various vitamins and minerals,
- HS medium (Hestrin Schramm),
- RAE medium with different content of ethanol and acetic acid (in general RAE medium comprises glucose, peptone, yeast extract, citric acid, Na2HPO4, acetic acid, ethanol and water),
or any combination of the above-mentioned additives, wherein the preferred additives are ethanol, acetic acid and/or RAE medium.

4. A process for obtaining the extract according to any of the preceding claims, **characterised in that** the process comprises the following steps:
a) lyophilisation of grape pomace, wherein the final moisture of pomace is from 5% to 15%, preferably 7%,
b) grinding of grape pomace lyophilised in step a) with any suitable device,
c) addition of water to grape pomace ground in step b), wherein the maximal final pomace concentration is 30%,
d) autoclaving the mixture obtained in step c), at a temperature of at least 121 °C, pressure 1.1 bar, and duration of 20 min with the possibility of time reduction for example for 5 to 10 min at higher temperature, for example, 134 °C,
e) separation of solid particles from the fluid part of the mixture autoclaved in step d) in order to obtain a liquid medium or an extract, respectively, which is suitable for use as a growth medium for microorganisms.

5. The process, according to the preceding claim, **characterised in that** lyophilisation is carried out from 24 hours to 72 hours at a temperature from -10 °C to -90 °C, and pressure 1 mbar, or any other combination of temperature and pressure, which allows direct sublimation of ice without any intermediate thawing.

6. The process, according to claim 4 or claim 5, **characterised in that** in step b) a pulveriser with 1000 rpm to 6000 rpm, from 1 min to 5 min, and sieve size from 0,08 µm to 1 µm is used.

7. The process, according to any claim from 4 to 6, **characterised in that** in step e) separation is achieved with decantation, sedimentation, centrifugation, filtration or draining, preferably with filtration through a 0.45 um PTFE filter.

8. The process, according to any claim from 4 to 7, **characterised in that** after step e) the extract is supplemented with additives selected in the group consisting of:
- ethanol,
- glycerol,
- mannitol,
- acetic acid or other acids,
- different carbon sources, such as glucose, sucrose, fructose, maltose, xylose, starch, mannose, glycerol and molasses,
- amino acids,
- yeast extract,
- peptone,
- various vitamins and minerals,
- HS medium (Hestrin Schramm),
- RAE medium with different content of ethanol and acetic acid (in general RAE medium comprises glucose, peptone, yeast extract, citric acid, Na2HPO4, acetic acid, ethanol and water),
or any combination of the above-mentioned additives, wherein the preferred additives are ethanol, acetic acid and/or RAE medium.

9. Use of the process and the extract according to any of the preceding claims in cultivation of microorganisms.

10. Use according to the preceding claim, wherein the extract is used for cultivation of bacteria selected in the group of bacterial genera *Acetobacter, Acidomonas, Alcaligenes, Agrobacterium, Ameyamaea, Asaia, Azotobacter, Bombella, Commensalibacter, Endobacter, Entomobacter, Gluconacetobacter, Komagataeibacter, Kozakia, Neoasaia, Neokomagataea, Nguyenibacter, Pseudomonas, Rhizobium, Saccharibacter, Salmonella, Sarcina, Swaminathania, Swingsia* and *Tanticharoenia.*

11. Use according to any claim from 9 to 10 in production of bacterial extracellular polysaccharides, preferably in production of cellulose.

12. A process for obtaining bacterial cellulose in a growth medium comprising the extract according to any claim from 1 to 3, wherein said process comprises the following steps:
a) preparation of the extract and optionally addition of additives in order to obtain a growth medium,
b) cultivation of the selected bacterial strain from any bacterial genera defined in claim 10 in a suitable amount of growth medium obtained in step a), at a temperature from 25 to 32 °C, preferably 30 °C, for at least 24 hours with shaking, followed by at least 3 days without shaking and at the same temperature to obtain a pellicle or cellulose membrane at the surface of the growth medium,
c) removal of the pellicle obtained in step b) from the medium and cooking in 0.5 M NaOH at a temperature of 80 °C for 30 minutes at constant mixing,
d) washing the cellulose membrane obtained in step c) in distilled water, and
e) drying the cellulose membrane washed in step d).

13. Use of bacterial cellulose obtained with the process according to the preceding claim in food industry, medicine, cosmetics, paper industry or textile industry.
